(19) Europäisches Patentamt
European Patent Office
Office européen des brevets

(11) **EP 4 087 946 B1**

(12) **EUROPEAN PATENT SPECIFICATION**

(45) Date of publication and mention
of the grant of the patent:
**13.03.2024  Bulletin 2024/11**

(21) Application number: **21701045.3**

(22) Date of filing: **06.01.2021**

(51) International Patent Classification (IPC):
***C12Q 1/6886*** (2018.01)

(52) Cooperative Patent Classification (CPC):
**C12Q 1/6886;** C12Q 2600/156

(86) International application number:
**PCT/EP2021/050136**

(87) International publication number:
**WO 2021/140125 (15.07.2021 Gazette 2021/28)**

(54) **METHODS OF DETERMINING PTEN COPY NUMBER**

VERFAHREN ZUR BESTIMMUNG DER PTEN-KOPIENZAHL

PROCÉDÉS DE DÉTERMINATION DU NOMBRE DE COPIES DE PTEN

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:  **07.01.2020  US 202062957913 P**

(43) Date of publication of application:
**16.11.2022  Bulletin 2022/46**

(73) Proprietor: **Astrazeneca AB
151 85 Södertälje (SE)**

(72) Inventors:
• **DOUGHERTY, Brian
Wilmington, Delaware 19850-5437 (US)**
• **LABROUSSE, Paul
Wilmington, Delaware 19850-5437 (US)**
• **STETSON, Daniel
Wilmington, Delaware 19850-5437 (US)**

(74) Representative: **AstraZeneca Intellectual Property
Eastbrook House
Shaftesbury Road
Cambridge CB2 8BF (GB)**

(56) References cited:
**CN-A- 110 616 251**

• **MOORE DAVID A. ET AL: "Accurate Detection of
Copy Number Changes in DNA Extracted from
Formalin-Fixed, Paraffin-Embedded Melanoma
Tissue Using Duplex Ratio Tests", THE JOURNAL
OF MOLECULAR DIAGNOSTICS, vol. 15, no. 5, 1
September 2013 (2013-09-01), pages 687-694,
XP055797266, ISSN: 1525-1578, DOI:
10.1016/j.jmoldx.2013.04.008**
• **WU XINGPING ET AL: "Simple quantitative PCR
analysis for allelic Pten loss in tumor
progression", ANALYTICAL BIOCHEMISTRY,
ACADEMIC PRESS, AMSTERDAM, NL, vol. 526,
18 March 2017 (2017-03-18), pages 50-57,
XP029971759, ISSN: 0003-2697, DOI:
10.1016/J.AB.2017.03.016**
• **BENJAMIN J. HINDSON ET AL:
"High-Throughput Droplet Digital PCR System
for Absolute Quantitation of DNA Copy Number",
ANALYTICAL CHEMISTRY, vol. 83, no. 22, 15
November 2011 (2011-11-15), pages 8604-8610,
XP055531826, US ISSN: 0003-2700, DOI:
10.1021/ac202028g**

## Description

### CLAIM OF PRIORITY

This application claims priority to provisional US 62/957,913, filed January 7, 2020, the contents of which are hereby incorporated by reference in their entirety.

### BACKGROUND

PTEN (phosphatase and tensin homologue deleted on chromosome ten) is a tumor suppressor that is mutated or deleted in a wide variety of human cancers, including cancers of the breast, prostate, endometrium, ovary, brain, skin, thyroid, lung, bladder and colon, as well as melanoma, glioblastoma, and lymphoma. Indeed, PTEN is one of the most commonly mutated tumor suppressor genes in cancer. PTEN is a phosphatase that modifies proteins and lipids. Unlike most phosphatases, the main substrates of PTEN are inositol phospholipids generated by the activation of the phosphoinositide 3 kinase (PI3K). The inositol phospholipids generated by PI3K lead to downstream activation of Akt, mTOR and ultimately cell survival and protein translation. Therefore, PTEN is an important negative regulator of PI3K/Akt signaling and plays a critical role in tumor suppression. During tumor development, mutations and deletions of PTEN occur that inactivate its enzymatic activity leading to increased cell proliferation and reduced cell death. The prevalence of PTEN mutations in cancer underscores the significance of identifying instances of genetic changes to PTEN.

A decrease in the PTEN copy number can reflect a loss of PTEN expression and/or enzymatic activity. Most methods of determining PTEN copy number consider only one locus at a time, and therefore may not accurately reflect deletions at loci other than the one investigated. Accordingly, improved methods of determining PTEN copy number are desirable.

WU XINGPING ET AL ("Simple quantitative PCR analysis for allelic Pten loss in tumor progression", ANALYTICAL BIOCHEMISTRY, vol. 526, 18 March 2017, pages 50-57) discloses a method to detect and quantify both PTEN mutation and allele-specific loss using allele-specific PCR analysis; one wildtype PTEN allele and one mutant PTEN allele (reference) are used to calculate the ratio. The same strategy is applied to digital PCR for comparison.

### SUMMARY

In one aspect, a method of determining PTEN copy number in a sample from a subject, includes: simultaneously amplifying three or more PTEN loci and two or more reference genes by digital PCR; calculating a ratio of PTEN amplification to reference gene amplification; and determining a PTEN copy number for the sample based on the ratio.

The ratio of PTEN amplification can be the ratio of: the concentration of all PTEN copies, divided by the number of PTEN loci amplified to: the concentration of all reference gene copies, divided by the number of reference genes amplified.

The ratio of PTEN amplification can be the ratio of: the concentration of copies of an individual PTEN locus to: the concentration of copies of an individual reference gene. In some embodiments, the method further includes determining the ratio of: the concentration of copies of each individual PTEN locus to: the concentration of copies of each individual reference gene.

The PTEN copy number can be the twice the ratio.

The three or more PTEN loci can include at least two of intron 2, intron 3, and intron 6. The three or more PTEN loci can include intron 2, intron 3, and intron 6, and optionally one or more additional PTEN loci.

In some embodiments, the method can further include determining the ratio of: the concentration of copies of a first reference gene to: the concentration of copies of a second reference gene.

In some embodiments, the method can further include diluting a portion of the sample with a corresponding sample of non-tumor origin.

The sample can include fresh frozen tumor tissue. The sample can includes cell free DNA. The sample can include formalin fixed parrafin embedded tumor or suspected tumor tissue.

The reference genes can include at least one of B3GNT2, ERCC3, ORST1, RUFY2, and CC2D 1B.

Other features, objects, and advantages will be apparent from the description and drawings, and from the claims.

### BRIEF DESCRIPTION OF THE DRAWINGS

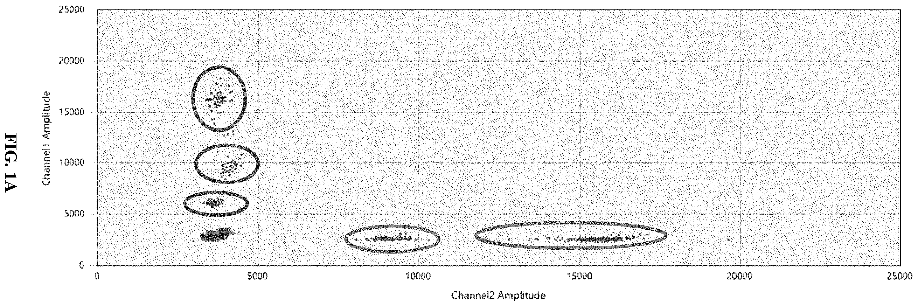

Fig. 1A. Example of cluster formation at 1ng input. Each circle at the bottom of graph represents droplets containing a unique reference gene locus, in this assay there are two targeted reference gene loci. Each of the three circles at the left of the graph represent droplets containing a PTEN locus. Droplets outside the circles contain targets from more than one locus.

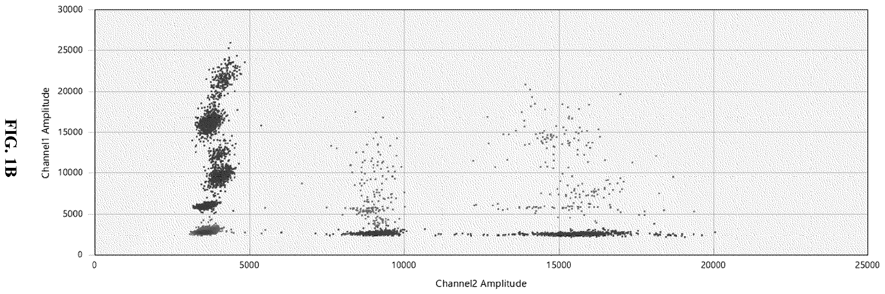

Fig. 1B. Example of cluster formation at a higher input (approximately 5ng).

**DETAILED DESCRIPTION**

Described herein are methods of determining PTEN copy number by simultaneously measuring more than one, e.g., two or more PTEN loci in a sample. By measuring multiple PTEN loci simultaneously, the present methods correctly identify instances of PTEN deletion that could otherwise go unnoticed, and can more precisely quantify the extent of PTEN deletion. The methods are also amenable to a variety of sample sources, including fresh frozen tissue, cell free DNA, and formalin fixed parrafin embedded (FFPE) tissue samples.

Patient samples derived from plasma have very low circulating free DNA (cfDNA) yields. Often, the entire DNA sample must be added to the Next Generation Sequencing (NGS) library reaction. In addition, DNA derived from the tumor (ctDNA) are often present in smaller fractions relative to cfDNA normally found in plasma samples. Due to these confounding factors, the detection of gene deletions in plasma becomes very challenging. It is beneficial to have orthogonal confirmation with a technology such as ddPCR to ensure accuracy of the copy number results detected by NGS.

In the methods of the invention, digital PCR (e.g., ddPCR) is used to simultaneously amplify more than one PTEN locus in a single experiment. The amplified material is then quantified, with the quantification being indicative of the copy number. Notably, the amplification and quantification occur locus-by-locus, so the copy number is measured independently at each locus. Thus, a change that affects only one locus can be distinguished from those that affect multiple loci.

In some embodiments, ddPCR is used to amplify and quantify PTEN loci. ddPCR allows simultaneous amplification of multiple loci with independent detection of each locus. Furthermore, one or more reference genes from the same sample is also simultaneously amplified and quantified with the PTEN loci. The reference genes are selected for having a known and stable copy number, providing a comparison for changes in PTEN copy number. In addition, discrepancies in the measured reference gene(s) may indicate a sample with high genomic instability.

In some embodiments, the quantification of amplified material is expressed as a concentration of copies of target DNA per unit volume. The measured concentrations of copies of PTEN loci and copies of reference genes can then be compared to arrive at a PTEN copy number (CN) in the sample.

In some embodiments, the total concentration of all PTEN loci is compared to the total concentration of the reference gene(s) to determine PTEN copy number. For example, when three PTEN loci and two reference genes are used,

$$\text{PTEN CN} = ((\text{PTEN Conc(copies/}\mu\text{L)/3)/(Reference gene Conc(copies/}\mu\text{L)/2))*2}$$

In some embodiments, the concentration of each PTEN locus is compared to the concentration of the reference gene(s) to determine PTEN copy number. In some embodiments, the concentration of each PTEN locus is compared to the concentration of each of the reference gene(s) to determine PTEN copy number. This technique provides independent measurements of copy number at each locus. For example, when three PTEN loci and two reference genes are used,

$$\text{PTEN (locus 1) CN} = ((\text{PTEN locus 1 Conc(copies/}\mu\text{L))/(Reference gene 1 Conc(copies/}\mu\text{L))*2}$$

$$\text{PTEN (locus 1) CN} = ((\text{PTEN locus 1 Conc(copies/}\mu\text{L))/(Reference gene 2 Conc(copies/}\mu\text{L))*2}$$

$$\text{PTEN (locus 2) CN} = ((\text{PTEN locus 2 Conc(copies/}\mu\text{L))/(Reference gene 1 Conc(copies/}\mu\text{L))*2}$$

$$\text{PTEN (locus 2) CN} = ((\text{PTEN locus 2 Conc(copies/}\mu\text{L))/(Reference gene 2 Conc(copies/}\mu\text{L))*2}$$

$$\text{PTEN (locus 3) CN} = ((\text{PTEN locus 3 Conc(copies/}\mu\text{L))/(Reference gene 1 Conc(copies/}\mu\text{L))*2}$$

$$PTEN\ (locus\ 3)\ CN = ((PTEN\ locus\ 3\ Conc(copies/\mu L))/(Reference\ gene\ 2\ Conc(copies/\mu L))*2$$

The concentration of copies of a first reference gene can be compared to the concentration of copies of one or more additional reference genes as an internal control, for example,

$$((Reference\ gene\ 1\ Conc(copies/\mu L))/(Reference\ gene\ 2\ Conc(copies/\mu L))*2$$

Samples where there is 30% or greater variation in the reference genes may be indicative of genomic instability. In such instances, the experiment can be repeated using different reference genes.

In some embodiments, the PTEN loci can include intron 2, intron 3, and intron 6. The PTEN loci can be two or more of intron 2, intron 3, and intron 6. References genes can include B3GNT2, ERCC3, OR5T1, RUFY2, and CC2D1B.

In some embodiments, samples are of tumor origin or suspected tumor origin. Samples of tumor origin or suspected tumor origin can optionally be compared to matched samples of non-tumor origin.

In some embodiments where the sample is FFPE tissue, controls reflecting different levels of FFPE damage (mild, moderate, severe) can be used to compare the tested sample.

## EXAMPLES

The following examples are illustrative and not intended to be limiting. Other embodiments are within the scope of the following claims.

## Methods

### Reference probe selection

Due to the potential for genomic instability in samples derived from cancer patients, an analysis was performed to identify several stable genes across different cancer indications, to utilize as normal references. Genomic stability was described as percent diploid (showing two copies) in copy number estimates derived from NGS testing. The genes were ranked by the percent diploid score to provide the best candidates to use for a reference gene. Several genes were tested and found to be suitable candidates as shown in Table 1.

Table 1. List of reference genes for use in PTEN copy number assay

| Gene | Chromosome |
|---|---|
| ERCC3 | 2 |
| OR5T1 | 11 |
| RUFY2 | 10 |
| B3GNT2 | 2 |
| CC2D1B | 1 |

### PTEN Probe Selection

Selection of the PTEN loci were determined by analyzing the copy number data available in the cBio database. Briefly, data from patients with confirmed PTEN loss were analyzed for probe selection. Regions with a high prevalence of PTEN loss were selected. As no one region was found to be deleted across all the patient data, a combination of three different loci were selected to maximize the clinical sensitivity of the assay. By comparing chromosomal coordinates of the targeted PTEN loci to the chromosomal coordinates of the segment files from the dataset, it was found that the design would potentially detect losses in 99.3% (731/736) of the reported samples within the data sets.

### Sample preparation

Dilute the test sample to 0.2ng/$\mu$L, which results in a total of 1ng input into the reaction. Using Poisson statistics, an input of 1ng should give a distribution where the majority of the droplets contain one target. Manipulating the input concentration to where each droplet contains only one target would theoretically enhance cluster separation, which would make analysis more efficient (see Figures 1A and 1B).

Figure 1A shows an example of cluster formation at 1ng input, note that the number of dual occupied droplets is minimal. Each blue circle represents droplets containing a unique PTEN locus, in this assay there are three targeted PTEN loci. Each green circle represents droplets containing a unique reference gene locus, in this assay there are two targeted reference gene loci. Droplets outside the circles contain targets from more than one locus. Figure 1B shows an example of cluster formation at a higher input (approximately 5ng), note that the increased number of dual occupied droplets, which can confound cluster delineation.

Preparation of the Master mix

Mix the components listed in Table 2 together and mix well by vortexing. The initial concentration is 9uM for both the forward and reverse primers, and SuM for each of the probes. The difference in the input volume for each of the PTEN loci (FAM channel) or the reference loci (HEX channel) is used to create differences in signal amplitude which creates separation between clusters. The total volume of the reaction is 22$\mu$L, but only 20$\mu$L is used for the droplet generation step (the remaining 2$\mu$L is considered dead volume).

Table 2. Master mix components for the PTEN copy number assay

| Component | Vol. per rxn ($\mu$L) | [Final] |
|---|---|---|
| 2$\times$ ddPCR Supermix for probes | 11 | 1$\times$ |
| PTEN locus 1 Primer/probe (FAM) | 1.5 | 1.4$\times$ |
| PTEN locus 2 Primer/probe (FAM) | 1.1 | 1.0$\times$ |
| PTEN locus 3 Primer/probe (FAM) | 0.55 | 0.5$\times$ |
| reference primer/probe R1 (HEX) | 1.1 | 1.0$\times$ |
| reference primer/probe R2 (HEX) | 0.77 | 0.7$\times$ |
| Water | 0.98 | NA |
| Sample (0.2ng/$\mu$L) | 5 | 1ng |

Droplet generation

Droplet generation is performed using the BIO-RAD Automated Droplet Generator. The manufacturer's recommendations are followed during this step. The output of this step is a 40$\mu$L volume containing approximately 20,000 droplets.

Amplification

After the test sample has been partitioned into droplets, amplification of the reaction was carried out. Table 3 outlines the thermal cycling parameters used for the PTEN copy number assay.

Table 3. Thermal cycling parameters for the PTEN copy number assay

| Cycling step | Temp (°C) | Time | Ramp rate | # cycles |
|---|---|---|---|---|
| Enzyme activation | 95 | 10 min | 2°C/sec | 1 |
| Denature | 94 | 30 sec | | 40 |
| Anneal/extend | 59 | 1 min | | |
| Enzyme Deactivation | 98 | 10 min | | |
| Hold | 4 | infinite | 1°C/sec | 1 |

Droplet reading

The individual droplets were interrogated using the BIO-RAD QX200 Droplet Reader. The data from these reads were used in result calling.

### Example 1

A tumor sample (FFPE) was assayed by ddPCR at three PTEN loci and two reference genes (B3GNT2 and ERCC3). The sample assayed at 100% concentration and repeated at dilutions with non-tumor material. Initial tumor cellularity was 70%. Table 4 summarizes the results:

Table 4: illustrative CN determination

| Dilution factor | Est. tumor % | CN using B3GNT2 | | | CN using ERCC3 | | | Aggregate CN est. |
|---|---|---|---|---|---|---|---|---|
| | | Intron 2 | Intron 6 | Intron 3 | Intron 2 | Intron 6 | Intron 3 | |
| - | 0.0 | 2.24 | 1.60 | 1.92 | 2.01 | 1.43 | 1.71 | 1.85 |
| 1 | 0.7 | 2.28 | 1.67 | 2.04 | 1.92 | 1.41 | 1.72 | 1.81 |
| 2.5 | 1.8 | 2.16 | 1.62 | 1.91 | 1.99 | 1.49 | 1.76 | 1.81 |
| 5 | 3.5 | 2.30 | 1.56 | 2.08 | 2.18 | 1.48 | 1.97 | 1.93 |
| 10 | 7.0 | 2.42 | 1.61 | 2.01 | 2.18 | 1.46 | 1.82 | 1.89 |
| 20 | 14.0 | 2.33 | 1.46* | 1.96 | 2.14 | 1.34* | 1.80 | 1.81 |
| 30 | 21.0 | 2.23 | 1.36* | 1.93 | 2.07 | 1.26* | 1.79 | 1.76** |
| 50 | 35.0 | 1.91* | 1.09* | 1.74* | 1.76* | 1.00* | 1.60* | 1.51* |
| 100 | 70.0 | 1.76* | 0.63* | 1.57* | 1.51* | 0.54* | 1.35* | 1.23* |
| *predicted PTEN loss **indeterminate | | | | | | | | |

### Example 2

Tissue and plasma samples from ER+ metastatic breast cancer patients were collected during a phase I trial of fulvestrant and capivasertib. For some patients, two samples were collected at different timepoints (designated 'baseline' and 'later' in the Table below).

The tissue samples were analyzed for PTEN status by Next Generation Sequencing (NGS), at the time of collection. The plasma (ctDNA) samples were analysed for PTEN status by low pass whole genome sequencing (LP-WGS) analysis performed with iCHOR CNA; and separately by ddPCR as described herein. Results are summarized in Table 5.

Table 5

| Patient ID | Sample time point | PTEN status (tissue NGS) | Tumor fraction (%) | PTEN status (ctDNA, iCHOR CNA LP-WGS) | PTEN status (ctDNA, ddPCR) |
|---|---|---|---|---|---|
| 951 | Baseline | PTENdel | 0 | PTENdel | PTENdel |
| | Later | PTENdel | 19 | PTENdel | PTENdel |
| 964 | Baseline | PTENdel | 19 | PTENdel | PTENdel |
| | Later | PTENdel | 31 | PTENdel | PTENdel |
| 991 | Baseline* | PTENdel | 17 | PTENdel | PTENdel |
| | Later* | PTENdel | 15 | PTEN copy neutral | PTENdel |
| 941 | Later | PTENdel | 11 | PTEN copy neutral | PTENdel |
| 879 | Later | PTENdel | 22 | PTEN copy neutral | PTENdel |
| 963 | Later | PTENdel | 19 | PTEN copy neutral | PTENdel |
| 962 | Baseline | PTENdel | 11 | PTEN copy neutral | PTENdel |
| *likely non-shedder | | | | | |

Table 5 shows that the determination of PTEN status by ctDNA LP-WGS miscategorized several patients as PTEN-copy neutral, disagreeing with the result obtained by NGS from tissue samples. In contrast, the ddPCR method uniformly agreed with the tissue sample results. The Tumor Fraction estimate was derived from the iCHOR CNA analysis of the LP-WGS data.

## Claims

1. A method of determining PTEN copy number in a sample from a subject, comprising:

   simultaneously amplifying three or more PTEN loci and two or more reference genes by digital PCR;
   calculating a ratio of PTEN amplification to reference gene amplification; and
   determining a PTEN copy number for the sample based on the ratio.

2. The method of claim 1, wherein the ratio of PTEN amplification is the ratio of:
   the concentration of all PTEN copies, divided by the number of PTEN loci amplified
   to:
   the concentration of all reference gene copies, divided by the number of reference genes amplified.

3. The method of claim 1, wherein the ratio of PTEN amplification is the ratio of:
   the concentration of copies of an individual PTEN locus
   to:
   the concentration of copies of an individual reference gene.

4. The method of claim 3, further comprising determining the ratio of:
   the concentration of copies of each individual PTEN locus
   to:
   the concentration of copies of each individual reference gene.

5. The method of any one of claims 1 to 4, wherein the PTEN copy number is the twice the ratio.

6. The method of any one of claims 1 to 5, wherein the three or more PTEN loci include at least two of intron 2, intron 3, and intron 6.

7. The method of claim 6, wherein the three or more PTEN loci include intron 2, intron 3, and intron 6, and optionally one or more additional PTEN loci.

8. The method of any one of claims 1 to 7, further comprising determining the ratio of:
   the concentration of copies of a first reference gene
   to:
   the concentration of copies of a second reference gene.

9. The method of any one of claims 1 to 8, further comprising diluting a portion of the sample with a corresponding sample of non-tumor origin.

10. The method of any one of claims 1 to 9, wherein the sample includes fresh frozen tumor tissue.

11. The method of any one of claims 1 to 9, wherein the sample includes cell free DNA.

12. The method of any one of claims 1 to 9, wherein the sample includes formalin fixed parrafin embedded tumor or suspected tumor tissue.

13. The method of any one of claims 1 to 12, wherein the reference genes include at least one of B3GNT2, ERCC3, OR5T1, RUFY2, and CC2D1B.

**Patentansprüche**

1.  Verfahren zur Bestimmung der PTEN-Kopienzahl in einer Probe von einem Individuum, umfassend:

    gleichzeitiges Amplifizieren von drei oder mehr PTEN-Loci und zwei oder mehr Referenzgenen mit digitaler PCR;
    Berechnen eines Verhältnisses von PTEN-Amplifikation zu Referenzgen-Amplifikation; und
    Bestimmen einer PTEN-Kopienzahl für die Probe anhand des Verhältnisses.

2.  Verfahren nach Anspruch 1, wobei das Verhältnis von PTEN-Amplifikation das Verhältnis von:
    der Konzentration aller PTEN-Kopien, geteilt durch die Anzahl amplifizierter PTEN-Loci,
    zu:
    der Konzentration aller Referenzgenkopien, geteilt durch die Anzahl amplifizierter Referenzgene, ist.

3.  Verfahren nach Anspruch 1, wobei das Verhältnis von PTEN-Amplifikation das Verhältnis von:
    der Konzentration von Kopien eines einzelnen PTEN-Locus zu:
    der Konzentration von Kopien eines einzelnen Referenzgens ist.

4.  Verfahren nach Anspruch 3, ferner umfassend Bestimmen des Verhältnisses von:
    der Konzentration von Kopien jedes einzelnen PTEN-Locus zu:
    der Konzentration von Kopien jedes einzelnen Referenzgens.

5.  Verfahren nach einem der Ansprüche 1 bis 4, wobei die PTEN-Kopienzahl das Zweifache des Verhältnisses beträgt.

6.  Verfahren nach einem der Ansprüche 1 bis 5, wobei die drei oder mehr PTEN-Loci wenigstens zwei von Intron 2, Intron 3 und Intron 6 umfassen.

7.  Verfahren nach Anspruch 6, wobei die drei oder mehr PTEN-Loci Intron 2, Intron 3 und Intron 6 und gegebenenfalls einen oder mehrere zusätzliche PTEN-Loci umfassen.

8.  Verfahren nach einem der Ansprüche 1 bis 7, ferner umfassend Bestimmen des Verhältnisses von:
    der Konzentration von Kopien eines ersten Referenzgens zu:
    der Konzentration von Kopien eines zweiten Referenzgens.

9.  Verfahren nach einem der Ansprüche 1 bis 8, ferner umfassend Verdünnen eines Teils der Probe mit einer entsprechenden nicht aus einem Tumor stammenden Probe.

10. Verfahren nach einem der Ansprüche 1 bis 9, wobei die Probe frisch gefrorenes Tumorgewebe umfasst.

11. Verfahren nach einem der Ansprüche 1 bis 9, wobei die Probe zellfreie DNA umfasst.

12. Verfahren nach einem der Ansprüche 1 bis 9, wobei die Probe formalinfixiertes, in Paraffin eingebettetes Tumor- oder vermutetes Tumorgewebe umfasst.

13. Verfahren nach einem der Ansprüche 1 bis 12, wobei die Referenzgene wenigstens eines von B3GNT2, ERCC3, OR5T1, RUFY2 und CC2D1B umfassen.

**Revendications**

1.  Procédé de détermination du nombre de copies de PTEN dans un échantillon provenant d'un sujet, comprenant :

    l'amplification simultanée de trois loci de PTEN ou plus et de deux gènes de référence ou plus par PCR numérique ;
    le calcul d'un rapport d'amplification de PTEN sur amplification de gènes de référence ; et
    la détermination d'un nombre de copies de PTEN pour l'échantillon sur la base du rapport.

2.  Procédé selon la revendication 1, le rapport d'amplification de PTEN étant le rapport de :
    la concentration de toutes les copies de PTEN, divisée par le nombre de loci de PTEN amplifiés sur :

la concentration de toutes les copies de gènes de référence, divisée par le nombre de gènes de référence amplifiés.

3. Procédé selon la revendication 1, le rapport d'amplification de PTEN étant le rapport de :
la concentration de copies d'un locus de PTEN individuel
sur :
la concentration de copies d'un gène de référence individuel.

4. Procédé selon la revendication 3, comprenant en outre la détermination du rapport de :
la concentration de copies de chaque locus de PTEN individuel
sur :
la concentration de copies de chaque gène de référence individuel.

5. Procédé selon l'une quelconque des revendications 1 à 4, le nombre de copies de PTEN étant le double du rapport.

6. Procédé selon l'une quelconque des revendications 1 à 5, les trois loci de PTEN ou plus comprenant au moins deux parmi l'intron 2, l'intron 3 et l'intron 6.

7. Procédé selon la revendication 6, les trois loci de PTEN ou plus comprenant l'intron 2, l'intron 3 et l'intron 6, et éventuellement un ou plusieurs loci de PTEN supplémentaires.

8. Procédé selon l'une quelconque des revendications 1 à 7, comprenant en outre la détermination du rapport de :
la concentration de copies d'un premier gène de référence
sur :
la concentration de copies d'un deuxième gène de référence.

9. Procédé selon l'une quelconque des revendications 1 à 8, comprenant en outre la déduction d'une partie de l'échantillon avec un échantillon correspondant d'origine non tumorale.

10. Procédé selon l'une quelconque des revendications 1 à 9, l'échantillon comprenant un tissu de tumeur fraîchement congelé.

11. Procédé selon l'une quelconque des revendications 1 à 9, l'échantillon comprenant de l'ADN acellulaire.

12. Procédé selon l'une quelconque des revendications 1 à 9, l'échantillon comprenant un tissu de tumeur ou de tumeur suspectée incorporé dans de la paraffine fixé à la formaline.

13. Procédé selon l'une quelconque des revendications 1 à 12, les gènes de référence comprenant au moins l'un parmi B3GNT2, ERCC3, OR5T1, RUFY2 et CC2D1B.

**FIG. 1A**

**FIG. 1B**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62957913 B

**Non-patent literature cited in the description**

- **WU XINGPING et al.** Simple quantitative PCR analysis for allelic Pten loss in tumor progression. *ANALYTICAL BIOCHEMISTRY,* 18 March 2017, vol. 526, 50-57